# EUROPEAN PATENT APPLICATION

(11) **EP 1 958 707 A1**
(43) Date of publication of application: **20.08.2008**
(21) Application number: 07290126.7
(22) Date of filing: 31.01.2007
(51) Int. Cl.: B05D 7/04, B05D 1/18, B05D 1/36, A61K 9/70, C08J 7/04

(54) **Mechanically responsive supported polyelectrolyte multilayer films**

(71) Applicant: INSERM (Institut National de la Santé et de la Recherche Medicale), 75013 Paris (FR)
(72) Inventor: Lavalle, Philippe, 67100 Strasbourg (FR); Hemmerle, Joseph, 67720 Weyersheim (FR); Mertz, Damien, 67800 Hoenheim (FR); Schaaf, Pierre, 67120 Molsheim (FR); Voegel, Jean Claude, 67210 Valff (FR)
(74) Representative: Colombet, Alain André

(57) **Abstract**

The invention concerns a mechanically responsive supported polyelectrolyte multilayer film, comprising:
- an elastic substrate, on which are alternately coated:
- at least one exponentially growing film; and
- at least one mechanically responsive barrier film.

It further concerns a process for its preparation and the use of such films notably for mechanically responsive mixing or delivery of active substances.

It further concerns dosage forms of a drug, biosensors and implants comprising such films.

## Description

The present invention concerns supported mechanically responsive polyelectrolyte multilayer films, their process of preparation and their uses.

The alternate deposition of polyanions and polycations on charged surfaces leads to the formation of nanostructured films called polyelectrolyte multilayers. The layer by layer deposition constitutes a formidable tool to functionalize surfaces and its potential applications range from optical coatings, filtration devices, self-supported membranes with highly enhanced Young's moduli, fuel cell membranes, biologically active membranes, drug release or biologically active coatings.

There exist two kinds of multilayers: those whose thickness and mass increase linearly with the number of deposition steps and those which grow exponentially. Linearly growing films are usually dense, nicely structured, a few tens of nanometers thick after deposition of ten layers. They generally act as impermeable barriers for the diffusion of polyelectrolytes and even of small ions.

Exponentially growing films are more gel-like, allow for the diffusion through the film section of polyelectrolytes and even proteins. They reach thicknesses up to a few micrometers within a few deposition steps. These films can be loaded with bioactive compounds and then act as reservoirs able to deliver such molecules to contacting cells.

Recently, multiple strata films composed of the alternation of linearly and exponentially growing films were designed. They allow the construction of multi-compartment architectures where the linearly growing films act as barriers (J.M. Garza, P. Schaaf, S. Muller, V. Ball, J.-F. Stoltz, J.-C. Voegel, and P. Lavalle, Langmuir 2004, 20, 7298-7302). The barriers prevent the diffusion of active compounds between two compartments, meaning from the upper compartment to the lower one, and inversely. These multi-compartment films are anticipated to be ideal architectures to create multi-functionalized surfaces with time scheduled activity or should allow to design new types of coatings where reactions between chemicals present in different compartments are induced by external stimuli. This, however, requires the opening of the barriers by the stimuli.

The opening by cellular degradation of biodegradable barriers has been described (J.M.Garza, N. Jessel, G. Ladam, V. Dupary, S. Muller, J.-F. Stoltz, P. Schaaf, J.-C. Voegel, and P. Lavalle, Langmuir 2005, 21, 12372-12377). Sequential or parallel multiagent delivery by degradation of the barrier films was also proposed (K.C. Wood, H.F. Chang, R.D. Batten, D.M. Lynn, P.T. Hammond, PNAS, 2006, 103,10207-10212).

Mechanically responsive multilayer polyelectrolyte films with surfaces switching from hydrophobic to hydrophilic substrate were also reported (J. Hemmerlé, V. Roucoules, G. Fleith, M. Nardin, V. Ball, P. Lavalle, P. Marie, J.-C. Voegel, P. Schaaf, Langmuir 2005, 21, 10328-10331). The first layer coated onto the prepared substrate was a linear growing film (PEI). These multilayer films behave differently than those reported here.

A main feature of the present invention lies in the discovery that the presence of an exponentially growing film as a first layer on an elastic substrate substantially modifies the behaviour of multilayer films. The exponentially growing film is believed to be sufficiently elastic to be deformed without breakage and to be capable of transmitting the mechanical stimuli to the whole multilayer film. Other layers present in the architecture may then reversibly form pores upon stretching of the substrate. Such multilayer films can thus act as mechanically responsive nano-sized barriers.

Therefore, according to a first aspect, the present invention concerns a mechanically responsive supported polyelectrolyte multilayer film, comprising:
- an elastic substrate on which are alternately coated :

- at least one exponentially growing film; and
- at least one mechanically responsive barrier film.

The term "mechanically responsive film" is meant to define a film which has at least some properties, such as its porosity, which change upon mechanical stimuli. A preferred mechanical stimuli is the stretching of the film above a critical value, expressed as the ratio of length to initial length I/I₀. The mechanical response of the films is reversible.

The term" barrier film" is a film which forms a barrier to the diffusion of species, except eventually for small ions like protons.

According to a preferred embodiment, the barrier films show a linear thickness increase upon coating. These films are generally dense and well ordered and films present a stratified structure, each polyelectrolyte layer interpenetrating only the neighboring ones.

Thus, the multilayer films described may be used as nano-valves. Upon application of a mechanical stress such as a slight elongation, the barrier layer form pores through which substances contained in the exponentially growing film may diffuse, thus opening the valve. When the application of the stress is stopped, the pores gradually heal and the barrier film again forbids diffusion, thus closing the valve.

Preferably, the barrier film is a polyelectrolyte film. A polyelectrolyte film is formed of alternate layers of polycationic and polyanionic compounds. The barrier film thus generally consists of one or more bilayers, each composed of one polycationic and one polyanionic layer.

In particular, the polycationic layer may comprise or consist of a polymer chosen from poly-(diallyldimethylammonium), poly(ethyleneimine), poly(allylamine).

The polyanionic layer may comprise or consist of a polymer chosen from poly(styrenesulfonate) or poly(vinylsulfate).

Therefore, the barrier layer preferably comprises at least one polymer chosen from the group consisting of poly(diallyldimethylammonium), poly(ethyleneimine), poly(allylamine), poly(styrenesulfonate), poly(vinylsulfate).

Poly(diallyldimethylammonium) / poly(styrenesulfonate) (PDADMA/PSS) multilayers are known to be linearly growing films with stratified architectures. They can be used as barrier strata when inserted between two exponentially growing layers and thus prevent PLL chain diffusion between different compartments.

The term "reservoir film" is understood to define an exponentially growing film. These films are generally ill-structured, gel-like film, which allow the diffusion of species.

The linear or exponential growth of films can be identified by measuring the incremental thickness for each deposited layer, using techniques known in the art such as the quartz crystal microbalance.

Generally, the exponentially growing film is a polyelectrolyte film. The polycationic layer may in particular comprise polymers chosen from the group consisting of polyaminoacids such as poly(lysine), poly(ornithin), chitosan. The polyanionic layer may in particular comprise polymers chosen from the group consisting of hyaluronic acid, poly(glutamic acid), alginate, chondroitin sulfate.

Therefore, the exponentially growing film preferably comprises at least one polymer chosen from the group consisting of poly-L-(lysine) (PLL), poly(ornithin), chitosan, hyaluronic acid (HA), poly(glutamic acid), alginate, chondroitin sulfate. PLL/HA multilayers are known to grow exponentially and PLL chains diffuse over the entire architecture. Preferred exponentially growing films are composed of (PLL/HA)ₙ films.

For some substances, notably of natural origin, one specific isomer is better available and thus preferred. This is the case for polyaminoacids, where the L-isomer is more common than the D-isomer. However, the different isomers are believed to act equal within the multilayer films, and both isomers are thus in principle equally appropriate.

Exponentially growing films may further comprise active substances that may be released upon opening of the barrier layer, as will be explained more in detail below.

Active substances can be introduced in these films by any known process, such as by diffusion or may be introduced directly upon the preparation of the multilayer film. It is possible to introduce active substances in the exponentially growing film for instance by dipping the multilayer film into a solution of the active substance or by using a solution containing the active substance to prepare the multilayer film.

The substrate is elastic in order to support the multilayer film adequately even upon mechanical solicitation, in particular stretching. Elastomers are good candidates as substrates, and among these, silicone has the further advantage of being rather inert. However, biocompatible and/or biodegradable substrates may be preferred for some applications.

The substrate may be submitted to different treatments before coating in a manner known in the art, for instance by grafting reactive groups in order to improve adhesion.

Under the term "elastic" is meant a material which at least mostly returns to its original form or position after being bent, compressed or stretched.

The supported multilayer film may further comprise a capping layer on top of the last exponentially growing film or barrier film. The capping layer is preferably equally a polyelectrolyte film, comprising the polymers cited above.

The exponentially growing films may comprise one or more active substances. Active substances can be chosen in particular from pharmaceutically active substances, coloured dyes, perfume, nanoparticles, cells or pesticides.

When the barrier layer becomes porous upon mechanical stimuli, notably by stretching the multilayer film above a critical value I/I₀, the active substances contained in the exponentially growing film(s) may diffuse throughout the layers or eventually outside the multilayer film.

Therefore, two distinct embodiments of the described multilayer films are envisaged, either for the delivery or the mixing of substances.

According to a first embodiment, a mechanically responsive barrier layer forms the external layer of the multilayer film. This external barrier layer may thus regulate the diffusion of active substances towards the outside of the multilayer film.

Such multilayer films may be useful in particular for the administration of pharmaceutically active substances, but also pharmaceutically active substances, coloured dyes, perfume, nanoparticles, cells or pesticides.

According to a second embodiment, the multilayer film comprises at least two exponentially growing layers, respectively separated by a mechanically responsive barrier layer. Mechanical stimuli of such multilayer films will open the barrier layer and allow the mixing of any substances contained in the different exponentially growing layers.

Such multilayer films may be useful in particular as molecular reactors for example when two components separated inside different exponentially growing films will be mixed once the stretching is applied, this will lead finally to a chemical reaction and to a new product.

It is of course also possible to combine both embodiments, that is a multilayer film comprising several reservoir films respectively separated by barrier layers and a barrier layer forming the external layer of the multilayer film.

Mechanically responsive supported polyelectrolyte multilayer films can thus be prepared by depositing polyelectrolyte multilayer films comprising barrier films and exponentially growing films onto an elastic substrate.

Thus, according to a second aspect, the invention concerns a process for the preparation of a multilayer film as described above, comprising the steps consisting of:
(i) providing an elastic substrate;
(ii) coating alternately an exponentially growing film and a mechanically responsive barrier film onto the substrate; and
(iii) eventually coating a capping layer onto the last layer.

Different techniques for layer-by-layer deposition of polyelectrolytes are known in the art. A preferred method for realizing the coating is dipping.

Preferably, the coated substrate is rinsed between each coating step with an appropriate solvent, generally water or a buffer solution.

According to a third aspect, the invention concerns the use of the described multilayer films for the mechanically responsive mixing and/or mechanically responsive delivery of active substances.

In that respect, the invention further aims at a dosage form comprising the multilayer film as described, in particular a patch or a plaster, for the administration of a drug.

Another useful application of the multilayer film is in the field of biosensors. Indeed, multilayer films as described could be used to detect a particular species upon mechanical stress. In this case, reservoir layers can be functionalized with a specific ligand of the molecules to be detected. Therefore, the invention also aims at a biosensor comprising a multilayer film as described.

Finally, another useful application of the invention is in the field of implants. Thus, according a last aspect, the invention aims at an implant, in particular an orthopaedic or a cardiovascular implant, comprising a multilayer film as described. In this application, the mere physiological motion may constitute the stimuli which lead to the desired multilayer film response and for example to a release of a specific biomolecules.

The invention will be described more in detail in the following examples and in the figures in annex, which show:

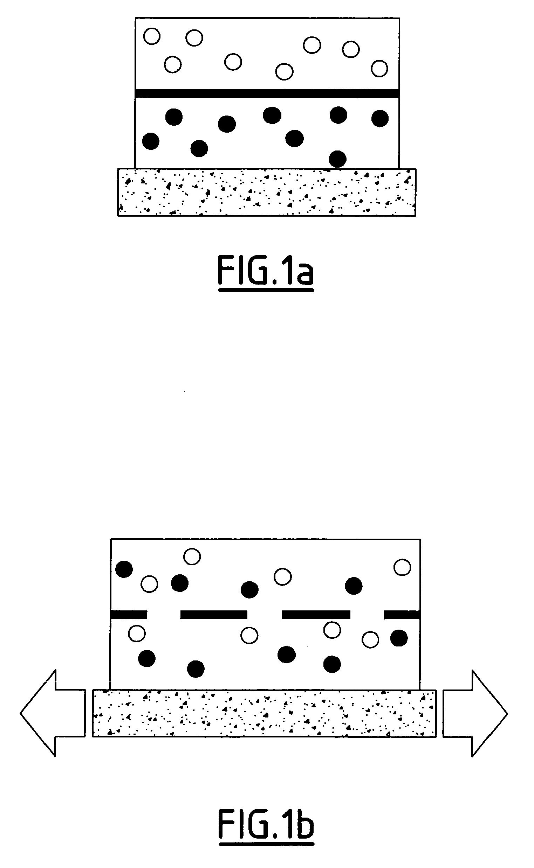

- Fig. 1 :: a schematic representation of a supported multilayer film according to a first embodiment of the invention;

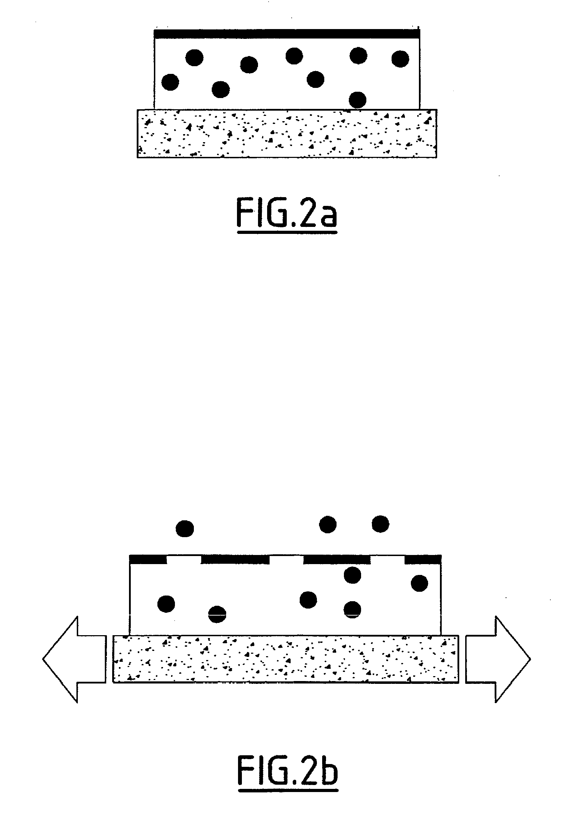

- Fig. 2 :: a schematic representation of a supported multilayer film according to a first embodiment of the invention;
- Fig.3 :: CLSM top view (x,y) images in the green channel of a (PLL/HA)₃₀/PLLRho/(HA/PLL)/(PSS/PDADMA)₅ multilayer film prepared according to example 1 brought in contact with a PLLFITC solution (PLLRho means poly(L-lysine) chains fluorescently labelled in red with Rhodamine and PLLFITC means poly(L-lysine) chains fluorescently labelled in green with Fluorescein Isothiocyanate) a) unstretched. b) stretched at I/I₀=1.5. The black arrow indicates the stretching direction. Image sizes are 47×47 µm².
- Fig. 4 :: Quartz Crystal Microbalance measurements monitoring the changes in the frequency shifts Δ*f* during the build-up of (PLL/HA)₅/PLL/(PSS/PDADMA)₅/HA/(PLL/HA)₃ multilayer films according to example 1.
- Fig. 5 :: Evolution of the film thickness of the film studied in Fig. 4 determined from the frequency changes and the dissipations measured at 15, 25 and 35 MHz by using the model of Voinava et al. (Voinova, M. V.; Rodahl, M.; Jonson, M.; Kasemo, B. Phys. Scr. 1999, 59 391-396).

### EXAMPLES

### EXAMPLE 1

### Preparation of a multilayer film with one reservoir layer

Poly(L-lysine) (PLL, Mw=5.57×10⁴ Da), poly(sodium 4-styrenesulfonate) (PSS, Mw=7.0×10⁴ Da), poly(diallydimethylammonium chloride) (PDADMA, Mw=2.0-3.5×10⁵ Da) were purchased from Sigma (St. Quentin Fallavier, France), and hyaluronic acid (HA, Mw=4.0×10⁵ Da) from Biolberica (Barcelona, Spain). Polyelectrolyte solutions were prepared by dissolution of adequate amounts of polyelectrolyte powders (or by dilution of commercial solution for PDADMA) in 0.15 M NaCl solution (pH = 6.5). The final concentrations of the polyelectrolyte solutions were 1 mg.mL⁻¹. All solutions were prepared using ultrapure water (Milli Q-plus system, Millipore) with a resistivity of 18.2 MΩ cm.

Multilayers were built with an automated dipping robot (Riegler & Kirstein GmbH, Berlin, Germany) on silicone sheets of 254 µm thick (Specialty Manufacturing Inc. SMI, Saginaw, Michigan, USA). Silicone sheets of 18x18 mm² were previously cleaned with ethanol and then extensively rinsed with water. Silicone substrates were first dipped in a PLL solution (polycation) for 10 min. Then, a rinsing step was performed by dipping the sheets for 5 min in 0.15M NaCl solution. The polyanion (HA) was then deposited in the same manner. The build-up process was pursued by the alternated deposition of PLL and HA. After deposition of n bilayers, the film is denoted (PLL/HA)*ₙ*. The same method was applied for PSS (polyanion) and PDADMA (polycation) depositions leading to (PDADMA/PSS)*ₘ* multilayers. The dye-conjugated polyelectrolytes were adsorbed in the same way at a certain stage of the build-up process.

On a bare silicone sheet, a (PLL/HA)₃₀ multilayer stratum is deposited as a primer coating, as previously described.

On top of the (PLL/HA)₃₀ multilayer, green fluorescently labeled poly(L-lysine) chains (PLLFITC) are deposited, as follows, leading to a complete labeling of the film section as previously described.

Fluorescein isothiocyanate labelled poly(L-lysine) (PLLFITC, Mw=3.0-7.0×10⁴ Da), was obtained from Sigma (St. Quentin Fallavier, France). Rhodamine Red™-X, succinimidyl ester (Invitrogen, France) was coupled to PLL. An appropriate amount of PLL (1 mg.mL⁻¹) was dissolved in a Na₂CO₃ solution (0.1 M, pH=8.5). Rhodamine (Rho) at 2 mg.mL⁻¹ was dissolved in DMSO (dimethylsulfoxyde). A ratio of 1 mg of rhodamine for 50 mg of PLL was used. The solutions were gently mixed at room temperature during 2 h. PLLRho was purified by dialysis during 3 days, and then the absence of free rhodamine in the solution was checked by UV spectroscopy according to the procedure described in Hermanson, G. T. In Bioconjugate techniques; Hermanson, G. T., Ed.; Academic Press: San Diego, 1996, pp 169-176.

The thickness of the entire multilayer film is around 5 µm.

This multilayer film was submitted to a longitudinal stretching under a confocal laser scanning microscope (CLSM) as described hereunder using a homemade stretching device.

This stretching device allowed to elongate silicone substrates in the longitudinal direction directly under the confocal microscope up to a stretching degree *I*/*I₀=*2.3. The stretching motion is achieved by a precision electric motor at a velocity of 0.74 mm.s⁻¹

The stretch degree is defined by the parameter I/I₀ where I₀ and I correspond to the initial length and to the stretched length of the silicone sheet respectively. Up to an elongation of 2 of the (PLL/HA)₃₀/PLLFITC film, no breakage or defect is visible. This demonstrates the strong adhesion of the PLL/HA multilayer on the silicone substrate and confirms its gel-like properties.

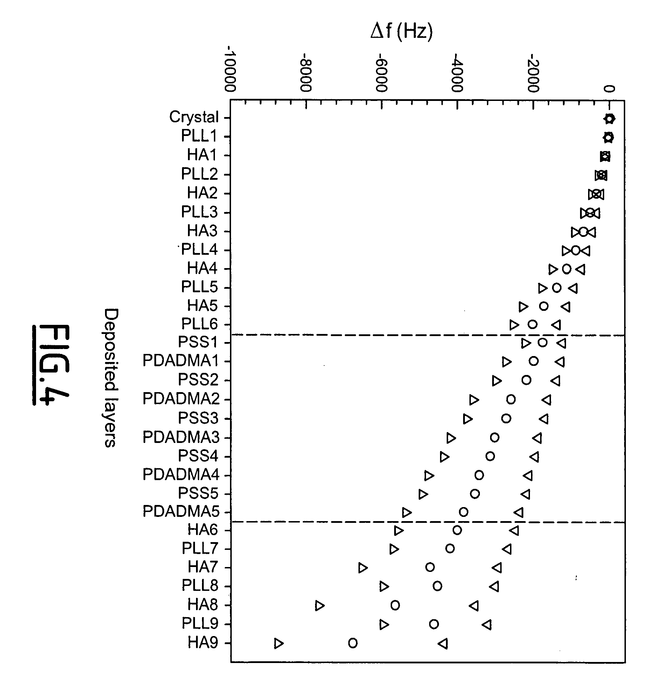

The (PLL/HA)₃₀/PLL multilayer was then capped with a (PSS/PDADMA)ₙ multilayer. The build-up of both strata multilayer films was checked with quartz crystal microbalance to control the regular change in frequency corresponding to a layer-by-layer deposition process. Moreover, the exponential growth of the first compartment can be clearly visualized when the growth is observed in Fig. 4 at 5 MHz, the more sensitive frequency. Then the PSS/PDADMA multilayers deposited on the first compartment exhibit the linear growth. This indicates the clear stratified structure of the compartment/barrier film.

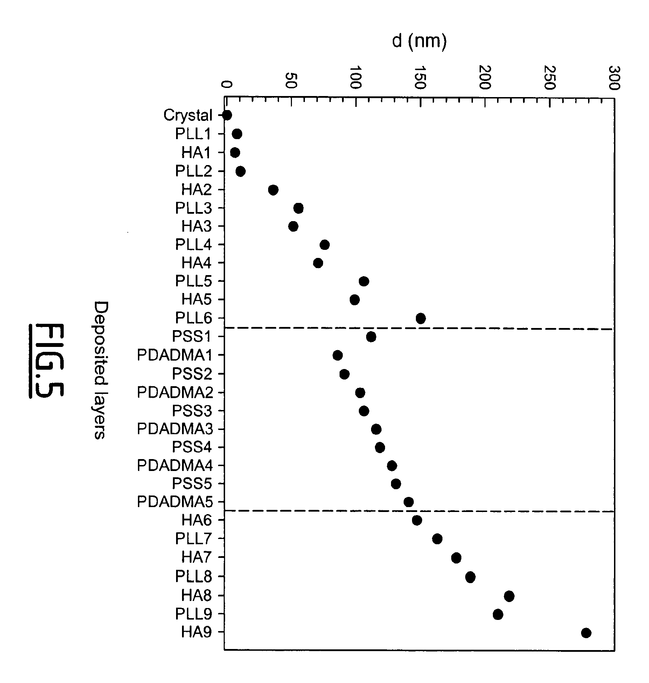

One observes from the results shown in Fig. 5 that the thickness of the (PLL/HA)₅/PLL film decreases during the deposition of the first PSS/PDADMA bilayer. This can be due to a rearrangement of the (PLL/HA)₅/PLL film when the nature of the polyelectrolytes deposited on top of the multilayer is changed. Such rearrangements could be the consequence of a change of the charge density on top of the film. It could also be due to a strong interaction of PSS and PDADMA respectively for PLL and HA. Some of these chains from the film beneath the barrier could thus be attracted towards the first PSS/PDADMA layer, leading to a thinning and densification of the (PLL/HA)₅/PLL film below.

Finally, from the evolution of the film thickness with the number of deposition steps one deduces that the thickness increment per PSS/PDADMA bilayer is 14 nm so that, for example, a (PSS/PDADMA)₅ barrier has a total thickness of 70 nm.

Such a (PSS/PDADMA) multilayer being used as a barrier for the first time, its efficiency to prevent diffusion of PLLFITC chains from the solution into the reservoir was checked with CLSM. This was performed by constructing a film with a PLL/HA compartment entirely labeled with PLLRho (red fluorescence from PLL chains labeled with Rhodamine succinimidyl ester) and capped with n=2, 5, 15 and 30 bilayers of PSS/PDADMA.

Then, the resulting film corresponding to (PLL/HA)₃₀/PLLRho/(HA/PLL)/(PSS/PDADMA)ₙ architecture was brought in contact with a PLLFITC solution (0.5 mg.mL⁻¹). No green color could be detected in the film section over more than 8 hours, proving that (PSS/PDADMA)*ₙ* multilayers act as barriers towards PLL diffusion. It was further verified that this multilayer barrier continues to prevent PLL diffusion when a (PLL/HA)₃₀ compartment is deposited on its top.

Next, the behavior of the (PLL/HA)₃₀/PLLRho/(HA/PLL) /(PSS/PDADMA)ₙ films under stretching was investigated for various architectures (n= 2, 5, 15 and 30). The films placed in PLLFITC solutions were observed with CLSM. By gradually increasing the stretching, a critical stretching degree was reached where the (PSS/PDADMA)ₙ films became inefficient to play the barrier role for preventing PLL chain diffusion from the solution to the underlying PLL/HA compartment. This critical stretching degree depends on the number n of bilayers constituting the barrier and increases with n.

The stretching degrees as a function of the number of bilayers n constituting the PSS/PDADMA barrier are summarized in Table 1. For of the silicone sheet higher than the critical stretching degree, PLLFITC chains from the solution diffuse into the (PLL/HA)₃₀ compartment. Once the barrier opens up for stretching degrees exceeding the critical value, PLL chains diffuse within a few minutes inside the (PLL/HA)₃₀ film. Below these values, the barriers ensure impermeability against PLL chains, when for higher values the barriers become inefficient to prevent PLL diffusion.

**Table 1: Critical stretching degrees as a function of the number of bilayers n**

| n | Critical stretching degree I/I₀ |
|---|---|
| 2 | 1.1 |
| 5 | 1.3-1.4 |
| 15 | 1.9-2.0 |
| 30 | >2.2 |

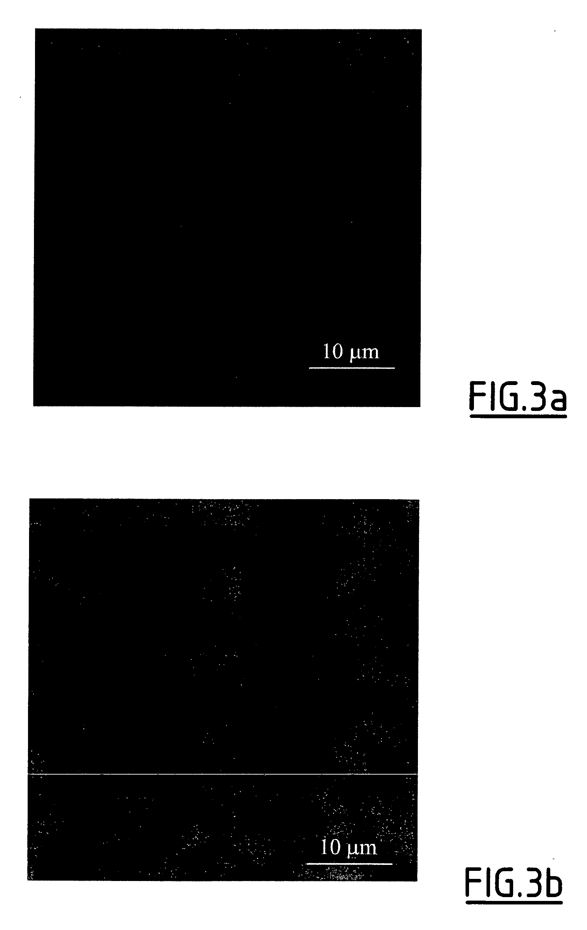

The final PLLFITC concentration in the film by far exceeds the PLLFITC concentration in the solution. Figure 3a illustrates the CLSM observation of the (PLL/HA)₃₀/PLLRho/(HA/PLL)/(PSS/PDADMA)₅ film incubated in a PLLFITC solution when no stretching is applied. The 2D view (x,y) at the film/solution interface depicts an homogenous film at the scale of the CLSM resolution without any defects in the structure. For stretching degrees smaller than I/I₀=1.4 interfaces show similar features with high homogeneity.

When the silicone sheets are stretched at I/I₀=1.5, which is above the critical stretching degree for n=5, small black spots corresponding to non-labeled pores appear on top of the film (Figure 3b). Their size is estimated to be of the order of a few hundreds of nanometers (from 100 nm to 1 µm).

These pores are only visualized when one focalizes at the upper part of the film which corresponds to the PSS/PDADMA strata. CLSM imaging of (x,y) planes inside the films shows uniform green fluorescence indicating that the pores are only located in the (PSS/PDADMA)ₙ barrier and do not extend down to the PLL/HA compartment.

The appearance of pores under stretching was also confirmed by Atomic Force Microscopy on a similar sample for a stretching degree of I/I₀=2. The characteristic sizes of the holes are of the order of 150-1500 nm, which is in accordance with the CLSM measurements. Atomic force imaging was performed using a D3000 Nanoscope IIIa (Veeco, Santa Barbara, CA). The apparatus operated in the tapping mode in dry condition. Cantilevers, with a spring constant of 0.05 N.m⁻¹ and a resonant frequency of 150 kHz ending with a silicon tip were used (Model MPP 12100, Veeco, Santa Barbara, CA).

### Confocal Laser Scanning Microscopy (CLSM)

Confocal laser scanning microscopy (CLSM) observations were carried out with a Zeiss LSM 510 microscope using a x40 (Zeiss Achroplan) objective and with 0.4 µm z-section intervals. FITC fluorescence was detected after excitation at λ=488 nm with a cut-off dichroïc mirror of 488nm and an emission band-pass filter of 505-530 nm (green emission). Rhodamine fluorescence was detected after excitation at λ=543 nm, dichroïc mirror of 543 nm, and an emission long pass filter of 585 nm (red emission). Virtual vertical sections can be visualized, hence allowing the determination of the thickness of the film. All the experiments are performed in liquid condition (NaCl 0.15 M, pH =6.5), and the multilayer films were never dried.

### Quartz Crystal Microbalance (QCM)

The build up process of the multilayered films was monitored in situ by quartz crystal microbalance-dissipation using the axial flow chamber QAFC 302 (QCM-D, D300, Q-Sense, Götenborg, Sweden). The QCM technique consists of measuring the resonance frequency (*f*) and the dissipation (D) of a quartz crystal induced by polyelectrolyte adsorption on the crystal, in comparison with the crystal in contact with NaCl solution. The crystal used here is coated with a 50nm thick SiO₂ film deposited by active sputter-coating. The quartz crystal is excited at its fundamental frequency (5 MHz), and the measurements are performed at the first, third, fifth and seventh overtones (denoted v) corresponding to 15, 25 and 35 MHz, respectively. Changes in the resonance frequency, Δ*f*, and in the dissipation factor, Δ*D*, during each adsorption step are measured. A shift in Δ*f* can be associated, in first approximation, with a variation of the mass adsorbed to the crystal. To characterize the film at a given step, only the frequencies and dissipations at the end of the rinsing steps following the exposure to either polycation or polyanion were taken into consideration. The experimental data (Δ*f* and Δ*D*) can be analyzed in the framework of the model developed by Voinova et al. (Voinova, M. V.; Rodahl, M.; Jonson, M.; Kasemo, B. Phys. Scr. 1999, 59 391-396).

Under the hypothesis that the film is a homogeneous and isotropic viscoelastic layer, a unique series of thickness values, *d,* is derived from Δ*f*/ν and Δ*D* corresponding to ν = 3, 5, and 7. The measurement methodology applied is disclosed in detail in Picart, C.; Lavalle, P.; Hubert, P.; Cuisinier, F. J. G.; Decher, G.; Schaaf, P.; Voegel, J.-C. Langmuir 2001, 17, 7414-7424).

For barriers constituted of less (n=2) or more (n=15 and 30) than n=5 PSS/PDADMA strata, the same conclusions can be drawn : below the critical stretching degree, the films remain homogenous and for stretching degrees higher than this critical value, pores appear in the barrier layer. The permeability of the barriers against PLL chains is related to the presence of these nanopores and can thus be finely controlled by the stretching degree applied on the film.

From the evolution of the film thickness with the number of deposition steps one deduces that the thickness increment per PSS/PDADMA bilayer is 14 nm so that, for example, a (PSS/PDADMA)₅ barrier has a total thickness of 70 nm.

In order to investigate the reversibility of the barrier opening, a (PLL/HA)₃₀/PLLRho/(HA/PLL)/(PSS/PDADMA)₅ film was stretched up to I/I0=1.8, corresponding to a value higher than the critical stretching degree, for 30 minutes and then brought back to its original non-stretched state. As soon as the film was brought back to its non stretched state, wrinkles with a periodicity about 5 µm and perpendicular to the stretching direction (y direction) appeared. These regular structures observed on top of the red labeled zone probably concern the reservoir/barrier interface. After maintaining the multilayer film for 30 minutes in a non-stretched state, wrinkles totally disappeared and the multilayer film recovered a flat and homogenous configuration. When this multilayer film was brought in contact with a PLLFITC solution, no diffusion of PLLFITC into the multilayer film could be detected over one hour, indicating that the (PSS/PDADMA)₅ stratum plays again the role of a PLL-tight barrier. These observations strongly suggest that the barrier opening and pore formation is a fully reversible process. These experiments also demonstrate that both the (HA/PLL)₃₀ and the (PSS/PDADMA)₅ multilayers behave as viscous fluid-like materials leading to healing processes of the barrier.

### EXAMPLE 2

### Preparation of a multilayer film with two compartments

A supported multilayer electrolyte film was prepared according to the example 1, except that two reservoir layers or compartments (PLL/HA)₃₀ were coated, separated by a (PSS/PDADMA)₅ layer. PLL from both (PLL/HA)₃₀ compartments were labeled, one with rhodamine, the other with fluorescein, leading to the following overall architecture : (PLL/HA)₃₀/PLLRho/(HA/PLL)/ (PSS/PDADMA)₅/(HA/PLL)₃₀/HA/PLLFITC.

When this multilayer film was stretched below I/I₀=1.4, no diffusion of PLL from one compartment to the other was observed during the whole observation period (four hours) and both compartments materialized by green and red areas are clearly separated.

The same conclusions can be drawn from observations one minute after applying a stretching of I/I₀=1.9, a value higher than the critical stretching degree. On the other hand, when the multilayer film was stretched to I/I₀=1.9 for 4 hours, a gradual diffusion of PLLFITC chains from the upper to the lower compartment and a gradual diffusion of PLLRho chains from the lower to the upper compartment occurred.

The (PSS/PDADMA)₅ barrier located in the middle of the film section becomes then strongly labeled with PLLFITC and PLLRho. The time scale of the diffusion of PLL chains from one compartment to the other lies in the order of 8 hours. After this time delay, the fluorescence through the film section is homogenous and the two compartments can no more be differentiated.

Moreover, as for the (PLL/HA)₃₀/PLLRho/(HA/PLL)/(PSS/PDADMA)₅ film, the appearance of pores in the barrier separating the two compartments, under stretching degrees exceeding the critical value (I/I0=1.9), were observed. Size and distribution of the pores in the film are very comparable to previous AFM observations on the multilayer capped by a barrier.

Thus the upper (PLL/HA)*ₙ* compartment exhibits only a minor influence on the behavior of the barrier under stretching. Once the multilayer film was brought back to its non-stretched state, wrinkles perpendicular to the stretching direction (y direction) also appeared as it was the case previously for the compartment/barrier system. These wrinkles also disappeared after a few tens of minutes.

Therefore, by alternating exponentially and linearly growing multilayers on a suitable substrate, it is possible to prepare multi-compartment films with nanosized barriers.

The nano-sized barriers can be opened and reversibly closed by mechanical stimuli, in particular by stretching. For the investigated system, the stretching induces the formation of pores in the barriers once a critical stretching degree is reached and consequently allows a diffusion process through the barrier of polyelectrolyte chains initially contained in the different compartments.

These multilayer films are thus able to respond, chemically or biologically, to mechanical stimuli.

In particular, the design of multilayer films containing several barriers whose critical stretching rates decrease gradually from the substrate towards the outer part of the architecture can be considered, leading to cascades of reactions controlled by adjusting the mechanical stretching rates.

## Claims

1. A mechanically responsive supported polyelectrolyte multilayer film, comprising:
- an elastic substrate, on which are alternately coated :
- at least one exponentially growing film; and
- at least one mechanically responsive barrier film.

2. A multilayer film according to claim 1, wherein the barrier film is a linear growing polyelectrolyte film.

3. A multilayer film according to claim 1 or 2, wherein the barrier layer preferably comprises at least one polymer chosen from the group consisting of poly (diallyldimethylammonium), poly(ethyleneimine), poly(allylamine), poly(styrenesulfonate), poly(vinylsulfate).

4. A multilayer film according to at least one of claims 1 to 3, wherein the exponentially growing film is a polyelectrolyte film.

5. A multilayer film according to at least one of claims 1 to 4, wherein the exponentially growing film comprises polymers chosen from the group consisting of poly-(lysine) (PLL), poly(ornithin), chitosan, hyaluronic acid (HA), poly(glutamic acid), alginate, chondroitin sulfate.

6. A multilayer film according to at least one of claims 1 to 5, wherein the substrate is made of silicone.

7. A multilayer film according to at least one of claims 1 to 6, wherein the multilayer film comprises at least two exponentially growing films, separated by a barrier film.

8. A multilayer film according to at least one of claims 1 to 7, comprising a capping layer on top of the last exponentially growing or barrier film.

9. A multilayer film according to at least one of claims 1 to 8, wherein the exponentially growing film comprises one or more active substances.

10. Process of preparation of a multilayer film according to at least one of claims 1 to 9, comprising the steps consisting of:
(i) providing an elastic substrate;
(ii) coating alternately a exponentially growing film and a mechanically responsive barrier film onto the substrate; and
(iii) eventually coating a capping layer onto the last layer.

11. Process according to claim 10, wherein the coating is realized by dipping.

12. Use of a multilayer film according to at least one of claims 1 to 9, comprising at least two exponentially growing films, for mechanically responsive mixing of substances.

13. Use of a multilayer film according to at least one of claims 1 to 9, for mechanically responsive delivery of active substances.

14. Dosage form for the administration of a drug comprising the multilayer film according to at least one of claims 1 to 9.

15. Dosage form according to claim 14, in form of a patch or a plaster.

16. Biosensor comprising a multilayer film according to at least one of claims 1 to 9.

17. Implant comprising a multilayer film according to at least one of claims 1 to 9.
